(19) Europäisches Patentamt
European Patent Office
Office européen des brevets

(11) **EP 0 841 060 B1**

(12) **EUROPÄISCHE PATENTSCHRIFT**

(45) Veröffentlichungstag und Bekanntmachung des Hinweises auf die Patenterteilung:
**24.04.2002 Patentblatt 2002/17**

(51) Int Cl.⁷: **A61K 7/06**, A61K 7/11

(21) Anmeldenummer: **97118298.5**

(22) Anmeldetag: **22.10.1997**

(54) **Haarbehandlungsmittel zur Festigung der Haare**

Hair-care composition for fixing the hair

Produit de soins capillaires pour la fixation des cheveux

(84) Benannte Vertragsstaaten:
**DE ES FR GB IT**

(30) Priorität: **07.11.1996 DE 19645909**

(43) Veröffentlichungstag der Anmeldung:
**13.05.1998 Patentblatt 1998/20**

(73) Patentinhaber: **Wella Aktiengesellschaft**
**64274 Darmstadt (DE)**

(72) Erfinder:
• **Schmenger, Jürgen**
**64331 Weiterstadt (DE)**
• **Wendel, Harald**
**64372 Ober-Ramstadt (DE)**
• **Franzke, Michael, Dr.**
**64380 Rossdorf (DE)**

(56) Entgegenhaltungen:
EP-A- 0 551 748          WO-A-94/08554
WO-A-96/00565          GB-A- 1 296 021
US-A- 5 053 218          US-A- 5 304 368

• **PATENT ABSTRACTS OF JAPAN vol. 009, no. 287 (C-314), 14. November 1985 (1985-11-14) & JP 60 132911 A (PENTEL KK), 16. Juli 1985 (1985-07-16)**

**Beschreibung**

Die Erfindung betrifft ein wäßrig-alkoholisches Mittel zur Festigung der Haare mit einem Gehalt an neutralisiertem Schellack in Kombination mit mindestens einem weiteren filmbildenden haarfestigenden Polymer.

Herkömmliche Mittel zur Festigung der Haare, insbesondere Haarsprays verwenden niedere Alkohole, wie beispielsweise Ethanol oder Isopropanol als Lösungsmittel für die filmbildenden, haarfestigenden Bestandteile. Diese niederen Alkohole gehören zu den flüchtigen organischen Verbindungen (volatile organic compounds, VOC), welche in der Diskussion stehen, zu der Luftverunreinigung beizutragen. Aus Umweltschutzgründen ist es daher erstrebenswert, Mittel zur Festigung der Haare mit einem reduzierten Anteil an flüchtigen organischen Verbindungen zur Verfügung zu stellen. Dies kann dadurch erfolgen, daß die niederen Alkohole ganz oder teilweise durch Wasser ersetzt werden. Wäßrige, alkoholfreie Haarsprays sind zwar bekannt, sie weisen aber Nachteile in ihren Anwendungseigenschaften, insbesondere bezuglich der benötigten langen Trocknungszeiten auf.

Mittel zur Festigung der Haare, in welchen ein Teil der niederen Alkohole durch Wasser ersetzt ist, sind ebenfalls bekannt. Derartige wäßrig-alkoholische Systeme weisen den Nachteil auf, daß sie schwer zu versprühen sind, da Alkohol/Wasser-Gemische mit einem Alkoholgehalt zwischen 30 und 60 Prozent ein Viskositätsmaximum durchlaufen. Dieses Viskositätsmaximum tritt bei Anwesenheit von üblichen, haarfestigenden Polymeren in noch verstärkterem Maße auf.

Es bestand daher die Aufgabe, ein Mittel zur Festigung der Haare zur Verfügung zu stellen, welches einen reduzierten Anteil an flüchtigen organischen Substanzen aufweist, sich gut versprühen läßt und gleichzeitig über gute Anwendungseigenschaften verfügt.

Es wurde nun gefunden, daß ein Zusatz von Schellack zu handelsüblichen synthetischen filmbildenden Polymeren eine Viskositätserniedrigung von wäßrig-alkoholischen Lösungen bewirkt, was sich außerordentlich vorteilhaft auf die Sprüheigenschaften dieser wäßrig-alkoholischen Systeme auswirkt. Durch Messung der zum Brechen des auf dem Haar gebildeten Filmes benötigten Kraft (Bruchkraftmessungen) wird bestätigt, daß sich die Festigerleistung bei einem teilweisen Austausch der Polymere durch Schellack nicht signifikant ändert.

Gegenstand der vorliegenden Erfindung ist ein Haarbehandlungsmittel mit einem Gehalt an

(A) zu 50 bis 100 % neutralisiertem Schellack,

(B) mindestens einem filmbildenden, haarfestigenden synthetischen Polymer,

(C) 10 bis 80 Gewichtsprozent, vorzugsweise 30 bis 70 Gewichtsprozent mindestens eines Alkohols mit 1 bis 4 Kohlenstoffatomen und

(D) 20 bis 90 Gewichtsprozent, vorzugsweise 30 bis 70 Gewichtsprozent Wasser.

Die Komponente (A) des erfindungsgemäßen Mittels wird vorzugsweise in einer Konzentration von 0,1 bis 10 Gewichtsprozent, besonders bevorzugt in einer Konzentration von 0,5 bis 5 Gewichtsprozent eingesetzt. Ein geeignetes Produkt wird beispielsweise unter der Handelsbezeichnung Schellack MHP 210 von der Firma Pennig, Hamburg/Deutschland oder in mit Borax vorneutralisierter Form unter der Handelsbezeichnung Spezialschellack SSB 63 HE-N von der Firma Stroever/Deutschland vertrieben.

Als Neutralisationsmittel für Schellack können beispielsweise primäre, sekundäre oder tertiare Amine, Aminoalkohole, Alkalihydroxide oder Ammoniak in Form von wäßrigen Ammoniaklösungen verwendet werden. Bevorzugte Neutralisationsmittel sind 2-Amino-2-methylpropanol, Triisopropylamin, Natriumhydroxid oder wäßrige Ammoniaklösung.

Die Komponente (B) wird vorzugsweise in einer Konzentration von 0,01 bis 20 Gewichtsprozent, besonders bevorzugt in einer Konzentration von 0,1 bis 10 Gewichtsprozent eingesetzt. Unter filmbildenden, haarfestigenden Polymeren sollen solche Polymere verstanden werden, welche bei Anwendung in 0,1 bis 10prozentiger alkoholisch-wäßriger Lösung in der Lage sind, auf dem Haar einen Polymerfilm abzuscheiden und auf diese Weise das Haar zu festigen.

Als Komponente (B) geeignete nichtionische Polymere sind Homopolymere des Vinylpyrrolidons, die beispielsweise unter den Handelsbezeichnungen Luviskol® K von der Firma BASF, Ludwigshafen/Deutschland oder PVP-K von der Firma ISP, Wayne/NJ, USA vertrieben werden, sowie Homopolymere des N-Vinylformamids, die beispielsweise unter der Handelsbezeichnung PVF von der Firma National Starch/USA vertrieben werden. Weitere geeignete synthetische filmbildende, nichtionische haarfestigende Polymere sind zum Beispiel Copolymerisate aus Vinylpyrrolidon und Vinylacetat, die beispielsweise unter den Handelsbezeichnungen LUVISKOL®VA von der Firma BASF, Ludwigshafen/Deutschland vertrieben werden; Terpolymere aus Vinylpyrrolidon, Vinylacetat und Vinylpropionat, die beispielsweise unter der Handelsbezeichnung LUVISKOL®VAP der Firma BASF, Ludwigshafen/-Deutschland vertrieben werden; Polyacrylamide, die beispielsweise unter den Handelsbezeichnungen AKYPOMINE®P 191 von der Firma CHEM-Y, Emmerich/-Deutschland oder SEPIGEL®305 von der Firma SEPPIC/USA vertrieben werden; Polyvinylalkohole, die beispielsweise unter den Handelsbezeichnungen ELVANOL® der Firma Du Pont oder VINOL® 523/540 der Firma Air Products/USA vertrieben werden, sowie Polyethylenglykole mit einem Molekulargewicht von 800 bis 20.000 g/mol, die beispielsweise unter den Handelsbezeichnungen LIPOXOL®1000 von der Firma HÜLS AG/ Deutschland, PLURACOL E 4000 von der Firma BASF/ Deutschland oder UPIWAX®20.000 von der Firma UPI vertrieben werden.

Als Komponente (B) geeignete synthetische anionische, filmbildende haarfestigende Polymere sind zum Beispiel t-Butylacrylat/Ethylacrylat/Methacrylsäure Copolymere, die zum Beispiel unter dem Handelsnamen Luvimer®100P von der Firma BASF, Ludwigshafen/Deutschland vertrieben wird. Weitere geeignete synthetische filmbildende, anionische Polymere sind zum Beispiel Crotonsäure-Vinylacetat-Copolymere, die beispielsweise in Form einer 60%igen Lösung in Isopropanol/Wasser unter der Handelsbezeichnung ARISTOFLEX® von der Firma HOECHST/ Deutschland vertrieben werden. Weitere geeignete anionische Polymere sind zum Beispiel Terpolymere aus Acrylsäure, Ethylacrylat und N-t-Butylacrylamid wie sie unter den Handelsnamen ULTRAHOLD 8 und ULTRAHOLD STRONG der Firma BASF, Ludwigshafen/Deutschland vertrieben werden.

Als Komponente (B) geeignete amphotere Polymere sind zum Beispiel Copolymere aus Octylacrylamid, t-Butylaminoethylmethacrylate und zwei oder mehr Monomeren bestehend aus Acrylsäure, Methacrylsäure oder deren Ester, wie sie zum Beispiel unter dem Handelsnamen AMPHOMER®28-4910 oder AMPHOMER LV-71 der Firma NATIONAL STARCH, USA erhältlich sind.

Von den kationischen Polymeren, die in dem erfindungsgemäßen Mittel als Komponente (B) enthalten sein können, sind zum Beispiel Polyvinylpyrrolidon/-Dimethylaminoethylmethacrylat-Polymere zu nennen, welche beispielsweise unter der Handelsbezeichnung Gafquat®755 N von der Firma Gaf Co., New York/USA vertrieben werden. Weitere kationische Polymere sind beispielsweise das von der Firma BASF AG, Ludwigshafen/Deutschland unter dem Handelsnamen LUVIQUAT®HM 550 vertriebene Copolymer aus Polyvinylpyrrolidon und Imidazoliminmethochlorid, das von der Firma Calgon, Pittsburgh/USA unter dem Handelsnamen Merquat®Plus 3300 vertriebene Terpolymer aus Dimethyldiallylammoniumchlorid, Natriumacrylat und Acrylamid, das von der Firma ISP/USA unter dem Handelsnamen Gaffix®VC 713 vertriebene Terpolymer aus Vinylpyrrolidon, Dimethylaminoethylmethacrylat und Vinylcaprolactam, das von der Firma Gaf unter dem Handelsnamen Gafquat®HS 100 vertriebene Vinylpyrrolidon/Methacrylamidopropyltrimethylammoniumchlorid-Copolymer und das von der Firma GOLDSCHMIDT, Essen/Deutschland unter dem Handelsnamen Abil® Quat 3272 vertriebene diquaternäre Polydimethylsiloxan.

Selbstverständlich kann das erfindungsgemäße Mittel auch weitere übliche kosmetische Zusätze, wie beispielsweise nichtfestigende nichtionische Polymere, wie zum Beispiel Polyethylenglykol mit einem Molekulargewicht von 600 g/mol, nichtfestigende anionische Polymere und nichtfestigende natürliche Polymere sowie deren Kombination in einer Menge von vorzugsweise 0,01-50 Gew.%, Parfümöle in einer Menge von vorzugsweise 0,01-5 Gew.%, Trübungsmittel wie zum Beispiel Ethylenglykoldistearat, in einer Menge von vorzugsweise 0,01-5 Gew.%; Netzmittel oder Emulgatoren aus den Klassen der anionischen, kationischen, amphoteren oder nichtionogenen oberflächenaktiven Substanzen, wie Fettalkoholsulfate, ethoxylierte Fettalkohole, Fettsäurealkanolamide, wie zum Beispiel die Ester der hydrierten Rizinusölfettsäuren in einer Menge von vorzugsweise 0,1-30 Gew.%; ferner Feuchthaltemittel, Farbstoffe, Lichtschutzmittel, Antioxidantien, Glanzgeber und Konservierungsstoffe in einer Menge von vorzugsweise 0,01-10 Gew.% enthalten. Weitere geeignete Zusätze sind chinesiches Balsamharz und Cellulosederivate, zum Beispiel Hydroxypropylcellulose, welche beispielsweise unter der Handelsbezeichnung NISSO SL® von der Firma Lehmann & Voss, Hamburg/Deutschland vertrieben wird.

Das erfindungsgemäße Mittel kann in verschiedenen Applikationsformen Anwendung finden, wie beispielsweise in Aerosolzubereitungen als Schaum oder als Spray, des weiteren aus Non-Aerosol, welches mittels einer Pumpe oder als "Pump and Spray" zum Einsatz kommt. Das erfindungsgemäße Mittel kann auch als färbendes oder pflegendes Haarbehandlungsmittel wie zum Beispiel als Farbfestiger oder Pflegefestiger formuliert sein.

Wenn das erfindungsgemäße Mittel in Form eines Aerosol-Haarsprays oder Aerosol-Haarlackes vorliegt, so enthält es zusätzlich 15 bis 85 Gewichtsprozent, bevorzugt 25 bis 75 Gewichtsprozent, eines Treibmittels und wird in einem Druckbehälter abgefüllt. Als Treibmittel sind beispielsweise niedere Alkane, wie zum Beispiel n-Butan, i-Butan und Propan, oder auch deren Gemische mit Dimethylether sowie ferner bei den in Betracht kommenden Drücken gasförmig vorliegende Treibmittel, wie beispielsweise $N_2$, $N_2O$ und $CO_2$ sowie Gemische der vorstehend genannten Treibmittel geeignet.

Das erfindungsgemäße Mittel zur Festigung der Haare kann auch in Form eines mit Hilfe einer geeigneten mechanisch betriebenen Sprühvorrichtung versprühbaren Non-Aerosol-Haarsprays oder eines Non-Aerosol-Haarlacks vorliegen.

Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtungen kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Unter Haarbehandlung soll die Behandlung des menschlichen Kopfhaares vor allem zum Zweck der Herstellung einer Frisur, zur Pflege oder zur Reinigung der Haare verstanden werden.

Des weiteres ist mit der erfindungsgemäßen Polymerkombination die Herstellung von Konzentraten moglich, welche einen verringerten Wassergehalt und/oder Lösungsmittelgehalt aufweisen. Die Konzentrate werden nach Transport und gegebenenfalls Lagerung durch Zugabe der erforderlichen Menge Wasser und/oder Lösungsmittel in ein anwendungsfähiges Haarbehandlungsmittel überführt.

Das erfindungsgemäße Mittel weist einen reduzierten Anteil flüchtiger organischer Substanzen auf, ist gut versprühbar und bewirkt eine gute Festigung der mit dem Mittel behandelten Haare.

Die nachfolgenden Beispiele sollen den Gegenstand der Erfindung näher erläutern. Der in den Beispielen verwendete Schellack ist mit Borax vorneutralisiert (Spezialschellack SS B 63 HE-N der Firma Stroever/Deutschland).

**Beispiel 1: Vergleichsversuche zur Viskosität bei einem Zusatz von Schellack zu Polymerlösungen**

Es wurden wäßrig-ethanolische Lösungen von reinem neutralisiertem Schellack, reinem t-Butylacrylat/-Ethylacrylat/Methacrylsaure Copolymer sowie einem Gemisch von neutralisiertem Schellack und t-Butylacrylat/Ethylacrylat/Methacrylsäure Copolymer mit einem wechselnden Ethanolgehalt hergestellt. Bei 25°C wurden die kinematischen Viskositäten der Lösungen gemessen. Als Meßgerät wurde ein Ubbelohde Kapillarviskosimeter (ViscoSystem AVS 500 der Firma Schott/Deutschland) vexendet. Die Ergebnisse sind in Tabelle 1 aufgeführt.

Tabelle 1:

| Viskositäten von wäßrig-ethanolischen Polymerlösungen bei 25°C in $mm^2 \cdot s^{-1}$ | | | |
|---|---|---|---|
| | Ethanolgehalt | | |
| | 30 Gew.% | 50 Gew.% | 70 Gew.% |
| 2 Gew.% Schellack, neutralisiert | 2,5 | 2,8 | 2,5 |
| 6 Gew.% t-Butylacry lat/Ethylacrylat/Meth acrylsäure Copolymer, neutralisiert | 17,6 | 18,7 | 14,9 |
| 2 Gew.% Schellack, neutralisiert + 6 Gew.% t-Butylacry lat/Ethylacrylat/ Meth acrylsäure Copolymer, neutralisiert | 17,2 | 18,0 | 13,5 |
| bei Additivität erwartete Viskosität | 20,1 | 21,5 | 17,4 |

Die Versuche zeigen, daß bei Zusatz der polymeren Substanz Schellack zu einer Polymerlösung keine Erhöhung, sondern überraschenderweise eine Verringerung der Viskosität auftritt.

**Beispiel 2: Vergleichsversuche zur Viskosität von Polymerlösungen bei einem teilweisen Austausch des Polymers durch Schellack**

Es wurden wäßrig-ethanolische Lösungen von verschiedenen Polymeren sowie Lösungen, in denen die Polymere teilweise durch mit Aminomethylpropanol vollständig neutralisiertem Schellack ersetzt wurden, hergestellt. Es wurden die Viskositäten unter den gleichen Bedingungen, wie bei Beispiel 1 beschrieben, gemessen. Die Ergebnisse sind in Tabelle 2 dargestellt.

Tabelle 2:

| Viskositäten von wäßrig-ethanolischen Polymerlösungen bei 25°C in $mm^2 \cdot s^{-1}$, Konzentrationsangaben in Gewichtsprozent | | | | | | | | | |
|---|---|---|---|---|---|---|---|---|---|
| Ethanol | 55 | 55 | 55 | 55 | 55 | 55 | 39 | 39 | 37 |
| Polymer A[1] | 6 | 4 | 6 | -- | -- | -- | -- | -- | -- |
| Polymer B[2] | -- | -- | -- | 6 | 4 | 6 | -- | -- | -- |

[1] Polymer A: t-Butylacrylat/Ethylacrylat/Methacrylsaure Copolymer, neutralisiert

[2] Polymer B: Octylacrylamid/t-Butylaminoethylmethacrylat/Acrylsäure Copolymer, neutralisiert

Tabelle 2: (fortgesetzt)

| Viskositäten von wäßrig-ethanolischen Polymerlösungen bei 25°C in $mm^2 \cdot s^{-1}$, Konzentrationsangaben in Gewichtsprozent | | | | | | | | | |
|---|---|---|---|---|---|---|---|---|---|
| Ethanol | 55 | 55 | 55 | 55 | 55 | 55 | 39 | 39 | 37 |
| Polymer C[3) | -- | -- | -- | -- | -- | -- | 12 | 8 | 12 |
| Schellack, neutralisiert | -- | 2 | 2 | -- | 2 | 2 | -- | 2 | 2 |
| Viskosität in $mm^2 \cdot s^{-1}$ | 18,6 | 11,5 | 17,1 | 25,0 | 14,9 | 22,4 | 8,8 | 6,1 | 8,4 |

[3) Polymer C: Vinylpyrrolidon/Vinylacetat Copolymer

Die Versuche zeigen, daß bei einem teilweisen Austausch von üblichen, haarfestigenden Polymeren durch Schellack eine deutliche Viskositätserniedrigung auftritt.

**Beispiel 3: VOC 55 % Haarspray Non Aerosol starker Halt**

| 1,5 g | Vinylpyrrolidon/Vinylacetat Copolymer |
|---|---|
| 1,0 g | Schellack |
| 0,2 g | Parfüm |
| 55,0 g | Ethanol |
| 42,3 g | Wasser |
| 100,0 g | |

**Beispiel 4: VOC 55 % Haarspray Non Aerosol starker Halt**

| 6,0 g | Octylacrylamid/t-Butylaminoethylmethacrylat/Acrylsäure Copolymer |
|---|---|
| 2,0 g | Schellack |
| 1,3 g | 2-Aminobutanol |
| 0,2 g | Parfüm |
| 55,0 g | Ethanol |
| 35,5 g | Wasser |
| 100,0 g | |

**Beispiel 5: VOC 55 % Haarspray Non Aerosol normaler Halt**

| 3,0 g | t-Butylacrylat/Ethylacrylat/methacrylsäure Copolymer |
|---|---|
| 1,5 g | Schellack |
| 0,8 g | 2-Amino-2-methylpropanol |
| 0,2 g | Parfüm |
| 20,0 g | Ethanol |
| 35,0 g | Dimethylether |
| 35,5 g | Wasser |
| 100,0 g | |

**Beispiel 6: Flüssigfestiger**

| | |
|---|---|
| 2,0 g | Vinylacetat/Crotonsäure Copolymer |
| 0,5 g | Schellack |
| 0,2 g | Parfüm |
| 55,0 g | Ethanol |
| 42,3 g | Wasser |
| 100,0 g | |

**Beispiel 7: Flüssigfestiger 2-Phasen**

| | |
|---|---|
| 10,0 g | Isododekan |
| 2,0 g | Vinylacetat/Crotonsäure Copoylmer |
| 1,0 g | Schellack |
| 0,3 g | Parfüm |
| 50,0 g | Ethanol |
| 36,7 g | Wasser |
| 100,0 g | |

**Beispiel 8 : Tönungsfestiger, versprühbar**

| | |
|---|---|
| 2,50 g | Vinylpyrrolidon/Vinylacetat Copolymer |
| 1,00 g | Schellack |
| 0,20 g | Hydriertes Rizinusöl, ethoxyliert mit 45 mol Ethylenoxid |
| 0,05 g | Acid Brown 4 (CI 14805) |
| 42,50 g | Ethanol |
| 53,75 g | Wasser |
| 100,0 g | |

**Patentansprüche**

1. Haarbehandlungsmittel mit einem Gehalt an

   (A) zu 50 bis 100 % neutralisiertem Schellack,
   (B) mindestens einem filmbildenden, haarfestigenden synthetischen Polymer,
   (C) 10 bis 80 Gewichtsprozent mindestens eines Alkohols mit 1 bis 4 Kohlenstoffatomen und
   (D) 20 bis 90 Gewichtsprozent Wasser.

2. Haarbehandlungsmittel nach Anspruch 1 in Form eines Aerosol-Haarsprays, enthaltend eine Zusammensetzung mit einem Gehalt an den Komponenten (A) bis (D) gemäß Anspruch 1 und zusätzlich zu dieser Zusammensetzung ein Treibmittel.

3. Mittel nach einhem der vorhergehenden Ansprüche, **dadurch gekennzeichnet, daß** Komponente (A) in einer Menge von 0,1 bis 10 Gewichtsprozent enthalten ist.

4. Mittel nach einem der vorhergehenden Ansprüche, **dadurch gekennzeichnet, daß** Komponente (A) neutralisiert ist mit einem Neutralisationsmittel, welches ausgewählt ist aus der Gruppe, bestehend aus primären, sekundären oder tertiären Aminen, Aminoalkoholen, Alkalihydroxiden und wässrigen Ammoniaklösungen.

**5.** Mittel nach einem der vorhergehenden Ansprüche, **dadurch gekennzeichnet, daß** Komponente (B) in einer Menge von 0,01 bis 20 Gewichtsprozent enthalten ist.

**6.** Mittel nach einem der vorhergehenden Ansprüche, **dadurch gekennzeichnet, daß** Komponente (C) in einer Menge von 30 bis 70 Gewichtsprozent enthalten ist.

**7.** Mittel nach einem der vorhergehenden Ansprüche, **dadurch gekennzeichnet, daß** Komponente (D) in einer Menge von 30 bis 70 Gewichtsprozent enthalten ist.

**8.** Mittel nach einem der vorhergehenden Ansprüche, **dadurch gekennzeichnet, daß** Komponente (B) ausgewählt ist aus der Gruppe bestehend aus Polyvinylpyrrolidon, Poly-N-Vinylformamid, Vinylpyrrolidon/Vinylacetat Copolymer, Vinylpyrrolidon/Vinylacetat/Vinylpropionat Copolymer, Polyacrylamid, Polyvinylalkohol, Polyethylenglykol mit einem Molekulargewicht von 800 bis 20.000 g/mol, t-Butylacrylat/Ethylacrylat/Methacrylsäure Copolymer, Crotonsäure/Vinylacetat Copolymer, Acrylsäure/Ethylacrylat/N-t-Butylacrylamid Copolymer, Copolymer aus Octylacrylamid, t-Butyl-aminoethylmethacrylat und zwei oder mehr Monomeren ausgewählt aus Acrylsäure, Methacrylsäure oder deren Estern, quaternisiertes Vinylpyrrolidon/Dimethylaminoethylmethacrylat Copolymer, Vinylpyrrolidon/ Imidazoliminmethochlorid Copolymer, Dimethyladiallylammoniumchlorid/Natriumacrylat/Acrylamid Copolymer, Vinylpyrrolidon/Dimethylaminoethylmethacrylat/Vinylcaprolactam Copolymer, Vinylpyrrolidon/Methacrylamidopropyltrimethylammoniumchlorid Copolymer und quaternisiertem Polydimethylsiloxan.

## Claims

**1.** Hair-treatment composition with a content of

(A) shellac, neutralized to 50 to 100%
(B) at least one film-forming, hair-setting synthetic polymer,
(C) 10 to 80 per cent by weight of at least one alcohol having 1 to 4 carbon atoms and
(D) 20 to 90 per cent by weight of water.

**2.** Hair-treatment composition according to Claim 1 in the form of an aerosol hair spray, comprising a composition with a content of components (A) to (D) according to Claim 1 and, in addition to this composition, a propellant.

**3.** Composition according to one of the preceding claims, **characterized in that** component (A) is present in an amount of from 0.1 to 10 per cent by weight.

**4.** Composition according to one of the preceding claims, **characterized in that** component (A) is neutralized with a neutralizing agent chosen from the group consisting of primary, secondary or tertiary amines, aminoalcohols, alkali metal hydroxides and aqueous ammonia solutions.

**5.** Composition according to one of the preceding claims, **characterized in that** component (B) is present in an amount of from 0.01 to 20 per cent by weight.

**6.** Composition according to one of the preceding claims, **characterized in that** component (C) is present in an amount of from 30 to 70 per cent by weight.

**7.** Composition according to one of the preceding claims, **characterized in that** component (D) is present in an amount of from 30 to 70 per cent by weight.

**8.** Composition according to one of the preceding claims, **characterized in that** component (B) is chosen from the group consisting of polyvinylpyrrolidone, poly-N-vinylformamide, vinylpyrrolidone/vinyl acetate copolymer, vinylpyrrolidone/vinyl acetate/vinyl propionate copolymer, polyacrylamide, polyvinyl alcohol, polyethylene glycol with a molecular weight of from 800 to 20 000 g/mol, t-butyl acrylate, ethyl acrylate, methacrylic acid copolymer, crotonic acid/vinyl acetate copolymer, acrylic acid/ethyl acrylate/N-t-butylacrylamide copolymer, copolymer of octylacrylamide, t-butylaminoethyl methacrylate and two or more monomers chosen from acrylic acid, methacrylic acid or esters thereof, quaternized vinylpyrrolidone/dimethylaminoethyl methacrylate copolymer, vinylpyrrolidone/imidazolimine methochloride copolymer, dimethyldiallylammonium chloride/sodium acrylate/acrylamide copolymer, vinylpyrrolidone/dimethylaminoethyl methacrylate/vinylcaprolactam copolymer, vinylpyrrolidone/methacrylamidopropyltrimethylammonium chloride copolymer and quaternized polydimethylsiloxane.

**Revendications**

1. Composition de soins capillaires contenant

   (A) un shellac neutralisé de 50 à 100 %,
   (B) au moins un polymère synthétique filmogène fixateur de cheveu,
   (C) de 10 à 80 pour cent en poids d'au moins un alcool ayant de 1 à 4 atomes de carbone et
   (D) 20 à 90 pour cent en poids d'eau.

2. Composition de soins capillaires selon la revendication 1, sous forme d'une laque capillaire en aérosol,comprenant une composition contenant les composants (A) à (D) selon la revendication 1 et un agent propulseur en plus de cette composition.

3. Composition selon l'une quelconque des revendications précédentes, **caractérisée en ce que** le composant (A) est présent en une quantité de 0,1 à 10 pour cent en poids.

4. Composition selon l'une quelconque des revendications précédentes, **caractérisée en ce que** le composant (A) est neutralisé avec un agent neutralisant qui est choisi parmi le groupe constitué d'amines primaires, secondaires ou tertiaires, d'amino-alcools, d'hydroxydes de métaux alcalins et de solutions aqueuses d'ammoniaque.

5. Composition selon l'une quelconque des revendications précédentes, **caractérisée en ce que** le composant (B) est présent en une quantité de 0,01 à 20 pour cent en poids.

6. Composition selon l'une quelconque des revendications précédentes, **caractérisée en ce que** le composant (C) est présent en une quantité de 30 à 70 pour cent en poids.

7. Composition selon l'une quelconque des revendications précédentes, **caractérisée en ce que** le composant (D) est présent en une quantité de 30 à 70 pour cent en poids.

8. Composition selon l'une quelconque des revendications précédentes, **caractérisée en ce que** le composant (B) est choisi parmi le groupe constitué d'une polyvinylpyrrolidone, d'un poly-N-vinylformamide, d'un copolymère vinylpyrrolidone/acétate de vinyle, d'un copolymère vinylpyrrolidone/acétate de vinyle/propionate de vinyle, d'un polyacrylamide, d'un alcool polyvinylique, d'un polyéthylèneglycol ayant un poids moléculaire de 800 à 20 000 g/mol, d'un copolymère acrylate de t-butyle/acrylate d'éthyle/acide méthacrylique, d'un copolymère acide crotonique/acétate de vinyle, d'un copolymère acide acrylique/acrylate d'éthyle/N-t-butylacrylamide, d'un copolymère d'octylacrylamide, de méthacrylate de t-butylaminoéthyle et de deux ou plusieurs monomères choisis parmi l'acide acrylique, l'acide méthacrylique ou leurs esters, d'un copolymère vinylpyrrolidone/méthacrylate de diméthylaminoéthyle quaternisé, d'un copolymère vinylpyrrolidone/méthochlorure d'imidazoline, d'un copolymère chlorure de diméthyldiallylammonium/acrylate de sodium/acrylamide, d'un copolymère vinylpyrrolidone/méthacrylate de diméthylaminoéthyle/vinylcaprolactame, d'un copolymère vinylpyrrolidone/chlorure de méthylacrylamidopropyltriméthylammonium et d'un polydiméthylsiloxane quaternisé.